# EUROPEAN PATENT APPLICATION

(11) **EP 3 443 980 A1**
(43) Date of publication of application: **20.02.2019**
(21) Application number: 17186278.2
(22) Date of filing: 15.08.2017
(51) Int. Cl.: A61K 38/26, A61P 43/00, A61P 3/04, A61P 27/00

(54) **COMPOUND FOR THE TREATMENT OF PATIENTS WITH LEPTIN-RESISTANT OBESITY, BARDET-BIEDL SYNDROME AND OTHER CILIOPATHIES**

(71) Applicant: Ciliocure Limited, Walliswood Dorking Surrey RH5 5RD (GB)
(72) Inventor: Döhler, Klaus-Dieter, 30657 Hannover (DE); Meyer, Markus, 30625 Hannover (DE)
(74) Representative: Wasner, Marita

(57) **Abstract**

The invention pertains to a peptide for the treatment of Bardet-Biedl syndrome and other ciliopathies, like primary ciliary dyskinesia, polycystic kidney and liver disease, nephronophthisis, Alström syndrome, Meckel-Gruber syndrome and some forms of retinal degeneration, like retinitis pigmentosa, rod-cone disease and retinal neuropathy, as well as the treatment of patients with leptin-resistant obesity.

SEQUENCE LISTING

<110> Ciliocure Limited

<120> Compound for the treatment of patients with leptin-resistant obesity,
Bardet-Biedl syndrome and other ciliopathies

<130> P10059EP00

<160> 14

<170> BiSSAP 1.3.6

<210> 1
<211> 31
<212> PRT
<213> Homo sapiens


<220>
<223> GLP-1(7-37): Amino acids 7-37 of GLP-1

<400> 1
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg Gly
            20                  25                  30

<210> 2
<211> 28
<212> PRT
<213> Artificial Sequence


<220>
<223> GLP-1(7-34)amide: Lys34 is amidated at C-terminus

<400> 2
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys
            20                  25

<210> 3
<211> 28
<212> PRT
<213> Artificial Sequence


<220>
<223> Curaglutide: Acetylated at His7 (N-terminus), amidated at Lys34
      (C-terminus)

<400> 3
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys
            20                  25

<210> 4
<211> 31
<212> PRT
<213> Artificial Sequence

<220>
<223> Liraglutide: Lysin K acylated with palmitic acid via glutamic
      acid E

<400> 4
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Arg Gly Arg Gly
            20                  25                  30

<210> 5
<211> 60
<212> PRT
<213> Artificial Sequence


<220>
<223> Albiglutide Part 1: twice the same sequence which is a derivative
      of GLP-1(7-36) wherein Ala8 is replaced by Gly

<400> 5
His Gly Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg His Gly
            20                  25                  30
Glu Gly Thr Phe Thr Ser Asp Val Ser Ser Tyr Leu Glu Gly Gln Ala
        35                  40                  45
Ala Lys Glu Phe Ile Ala Trp Leu Val Lys Gly Arg
    50                  55                  60

<210> 6
<211> 585
<212> PRT
<213> Artificial Sequence


<220>
<223> Albiglutide Part 2: Albumin part of Albiglutide

<400> 6

<210> 7
<211> 645
<212> PRT
<213> Artificial Sequence

<220>
<223> Albiglutide: complete sequence of Albiglutide

<400> 7
His Gly Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg His Gly
            20                  25                  30
Glu Gly Thr Phe Thr Ser Asp Val Ser Ser Tyr Leu Glu Gly Gln Ala
        35                  40                  45
Ala Lys Glu Phe Ile Ala Trp Leu Val Lys Gly Arg Asp Ala His Lys
    50                  55                  60
Ser Glu Val Ala His Arg Phe Lys Asp Leu Gly Glu Glu Asn Phe Lys
65                  70                  75                  80
Ala Leu Val Leu Ile Ala Phe Ala Gln Tyr Leu Gln Gln Cys Pro Phe
                85                  90                  95
Glu Asp His Val Lys Leu Val Asn Glu Val Thr Glu Phe Ala Lys Thr
            100                 105                 110
Cys Val Ala Asp Glu Ser Ala Glu Asn Cys Asp Lys Ser Leu His Thr
        115                 120                 125
Leu Phe Gly Asp Lys Leu Cys Thr Val Ala Thr Leu Arg Glu Thr Tyr
    130                 135                 140
Gly Glu Met Ala Asp Cys Cys Ala Lys Gln Glu Pro Glu Arg Asn Glu
145                 150                 155                 160
Cys Phe Leu Gln His Lys Asp Asp Asn Pro Asn Leu Pro Arg Leu Val
                165                 170                 175
Arg Pro Glu Val Asp Val Met Cys Thr Ala Phe His Asp Asn Glu Glu
            180                 185                 190
Thr Phe Leu Lys Lys Tyr Leu Tyr Glu Ile Ala Arg Arg His Pro Tyr
        195                 200                 205
Phe Tyr Ala Pro Glu Leu Leu Phe Phe Ala Lys Arg Tyr Lys Ala Ala
    210                 215                 220
Phe Thr Glu Cys Cys Gln Ala Ala Asp Lys Ala Ala Cys Leu Leu Pro
225                 230                 235                 240
Lys Leu Asp Glu Leu Arg Asp Glu Gly Lys Ala Ser Ser Ala Lys Gln
                245                 250                 255
Arg Leu Lys Cys Ala Ser Leu Gln Lys Phe Gly Glu Arg Ala Phe Lys
            260                 265                 270
Ala Trp Ala Val Ala Arg Leu Ser Gln Arg Phe Pro Lys Ala Glu Phe
        275                 280                 285
Ala Glu Val Ser Lys Leu Val Thr Asp Leu Thr Lys Val His Thr Glu
    290                 295                 300
Cys Cys His Gly Asp Leu Leu Glu Cys Ala Asp Asp Arg Ala Asp Leu
305                 310                 315                 320
Ala Lys Tyr Ile Cys Glu Asn Gln Asp Ser Ile Ser Ser Lys Leu Lys
                325                 330                 335
Glu Cys Cys Glu Lys Pro Leu Leu Glu Lys Ser His Cys Ile Ala Glu
            340                 345                 350
Val Glu Asn Asp Glu Met Pro Ala Asp Leu Pro Ser Leu Ala Ala Asp
        355                 360                 365
Phe Val Glu Ser Lys Asp Val Cys Lys Asn Tyr Ala Glu Ala Lys Asp
    370                 375                 380
Val Phe Leu Gly Met Phe Leu Tyr Glu Tyr Ala Arg Arg His Pro Asp
385                 390                 395                 400
Tyr Ser Val Val Leu Leu Leu Arg Leu Ala Lys Thr Tyr Glu Thr Thr
                405                 410                 415
Leu Glu Lys Cys Cys Ala Ala Ala Asp Pro His Glu Cys Tyr Ala Lys
            420                 425                 430
Val Phe Asp Glu Phe Lys Pro Leu Val Glu Glu Pro Gln Asn Leu Ile
        435                 440                 445
Lys Gln Asn Cys Glu Leu Phe Glu Gln Leu Gly Glu Tyr Lys Phe Gln
    450                 455                 460
Asn Ala Leu Leu Val Arg Tyr Thr Lys Lys Val Pro Gln Val Ser Thr
465                 470                 475                 480
Pro Thr Leu Val Glu Val Ser Arg Asn Leu Gly Lys Val Gly Ser Lys
                485                 490                 495

Cys Cys Lys His Pro Glu Ala Lys Arg Met Pro Cys Ala Glu Asp Tyr
                500                 505                 510
Leu Ser Val Val Leu Asn Gln Leu Cys Val Leu His Glu Lys Thr Pro
        515                 520                 525
Val Ser Asp Arg Val Thr Lys Cys Cys Thr Glu Ser Leu Val Asn Arg
    530                 535                 540
Arg Pro Cys Phe Ser Ala Leu Glu Val Asp Glu Thr Tyr Val Pro Lys
545                 550                 555                 560
Glu Phe Asn Ala Glu Thr Phe Thr Phe His Ala Asp Ile Cys Thr Leu
                565                 570                 575
Ser Glu Lys Glu Arg Gln Ile Lys Lys Gln Thr Ala Leu Val Glu Leu
            580                 585                 590
Val Lys His Lys Pro Lys Ala Thr Lys Glu Gln Leu Lys Ala Val Met
        595                 600                 605
Asp Asp Phe Ala Ala Phe Val Glu Lys Cys Cys Lys Ala Asp Asp Lys
    610                 615                 620
Glu Thr Cys Phe Ala Glu Glu Gly Lys Lys Leu Val Ala Ala Ser Gln
625                 630                 635                 640
Ala Ala Leu Gly Leu
                645

<210> 8
<211> 29
<212> PRT
<213> Artificial Sequence

<220>
<223> Dulaglutide Part 1: Derivative of GLP-1(7-35) wherein Ala8 is
      repalced by Gly and Gly22 is replaced by Glu

<400> 8
His Gly Glu Gly Thr Phe Thr Ser Asp Val Ser Ser Tyr Leu Glu Glu
1               5                   10                  15
Gln Ala Ala Lys Glu Phe Ile Ala Trp Leu Val Lys Gly
            20                  25

<210> 9
<211> 246
<212> PRT
<213> Artificial Sequence

<220>
<223> Dulaglutide Part 2: Second part of Dulaglutide

<400> 9
Gly Gly Gly Gly Gly Gly Ser Gly Gly Gly Gly Ser Gly Gly Gly Gly
1               5                   10                  15
Ser Ala Glu Ser Lys Tyr Gly Pro Pro Cys Pro Pro Cys Pro Ala Pro
            20                  25                  30
Glu Ala Ala Gly Gly Pro Ser Val Phe Leu Phe Pro Pro Lys Pro Lys
        35                  40                  45
Asp Thr Leu Met Ile Ser Arg Thr Pro Glu Val Thr Cys Val Val Val
    50                  55                  60
Asp Val Ser Gln Glu Asp Pro Glu Val Gln Phe Asn Trp Tyr Val Asp
65                  70                  75                  80
Gly Val Glu Val His Asn Ala Lys Thr Lys Pro Arg Glu Glu Gln Phe
                85                  90                  95
Asn Ser Thr Tyr Arg Val Val Ser Val Leu Thr Val Leu His Gln Asp
            100                 105                 110
Trp Leu Asn Gly Lys Glu Tyr Lys Cys Lys Val Ser Asn Lys Gly Leu
        115                 120                 125

Pro Ser Ser Ile Glu Lys Thr Ile Ser Lys Ala Lys Gly Gln Pro Arg
    130                 135                 140
Glu Pro Gln Val Tyr Thr Leu Pro Pro Ser Gln Glu Glu Met Thr Lys
145                 150                 155                 160
Asn Gln Val Ser Leu Thr Cys Leu Val Lys Gly Phe Tyr Pro Ser Asp
                165                 170                 175
Ile Ala Val Glu Trp Glu Ser Asn Gly Gln Pro Glu Asn Asn Tyr Lys
            180                 185                 190
Thr Thr Pro Pro Val Leu Asp Ser Asp Gly Ser Phe Phe Leu Tyr Ser
        195                 200                 205
Arg Leu Thr Val Asp Lys Ser Arg Trp Gln Glu Gly Asn Val Phe Ser
    210                 215                 220
Cys Ser Val Met His Glu Ala Leu His Asn His Tyr Thr Gln Lys Ser
225                 230                 235                 240
Leu Ser Leu Ser Leu Gly
                245

<210> 10
<211> 275
<212> PRT
<213> Artificial Sequence

<220>
<223> Dulaglutide: Complete sequence of Dulaglutide; Disulfide bridge:
      90-150; 196-254. Dimer: 55; 58

<400> 10
His Gly Glu Gly Thr Phe Thr Ser Asp Val Ser Ser Tyr Leu Glu Glu
1               5                   10                  15
Gln Ala Ala Lys Glu Phe Ile Ala Trp Leu Val Lys Gly Gly Gly Gly
            20                  25                  30
Gly Gly Gly Ser Gly Gly Gly Gly Ser Gly Gly Gly Gly Ser Ala Glu
        35                  40                  45
Ser Lys Tyr Gly Pro Pro Cys Pro Pro Cys Pro Ala Pro Glu Ala Ala
    50                  55                  60
Gly Gly Pro Ser Val Phe Leu Phe Pro Pro Lys Pro Lys Asp Thr Leu
65                  70                  75                  80
Met Ile Ser Arg Thr Pro Glu Val Thr Cys Val Val Val Asp Val Ser
                85                  90                  95
Gln Glu Asp Pro Glu Val Gln Phe Asn Trp Tyr Val Asp Gly Val Glu
            100                 105                 110
Val His Asn Ala Lys Thr Lys Pro Arg Glu Glu Gln Phe Asn Ser Thr
        115                 120                 125
Tyr Arg Val Val Ser Val Leu Thr Val Leu His Gln Asp Trp Leu Asn
    130                 135                 140
Gly Lys Glu Tyr Lys Cys Lys Val Ser Asn Lys Gly Leu Pro Ser Ser
145                 150                 155                 160
Ile Glu Lys Thr Ile Ser Lys Ala Lys Gly Gln Pro Arg Glu Pro Gln
                165                 170                 175
Val Tyr Thr Leu Pro Pro Ser Gln Glu Glu Met Thr Lys Asn Gln Val
            180                 185                 190
Ser Leu Thr Cys Leu Val Lys Gly Phe Tyr Pro Ser Asp Ile Ala Val
        195                 200                 205
Glu Trp Glu Ser Asn Gly Gln Pro Glu Asn Asn Tyr Lys Thr Thr Pro
    210                 215                 220
Pro Val Leu Asp Ser Asp Gly Ser Phe Phe Leu Tyr Ser Arg Leu Thr
225                 230                 235                 240
Val Asp Lys Ser Arg Trp Gln Glu Gly Asn Val Phe Ser Cys Ser Val
                245                 250                 255
Met His Glu Ala Leu His Asn His Tyr Thr Gln Lys Ser Leu Ser Leu
            260                 265                 270
Ser Leu Gly

        275

<210> 11
<211> 30
<212> PRT
<213> Artificial Sequence

<220>
<223> Taspoglutide: GLP-1 (7-36) wherein Ala8 is replaced by Aib, Gly35
      is replaced by Aib

<400> 11
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg
            20                  25                  30

<210> 12
<211> 39
<212> PRT
<213> Artificial Sequence

<220>
<223> Exendin-4: Ser39 is amidated at C-terminus

<400> 12
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210> 13
<211> 39
<212> PRT
<213> Artificial Sequence

<220>
<223> Exenatide: Ser39 is amidated at C-terminus

<400> 13
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210> 14
<211> 44
<212> PRT
<213> Artificial Sequence

<220>
<223> Lixisenatide: Lys44 is amidated at C-terminus

<400> 14
His Gly Glu Gly Thr Phe Thr Ser Asp Leu Ser Lys Gln Met Glu Glu

1                   5                   10                  15
Glu Ala Val Arg Leu Phe Ile Glu Trp Leu Lys Asn Gly Gly Pro Ser
            20                  25                  30
Ser Gly Ala Pro Pro Ser Lys Lys Lys Lys Lys Lys
        35                  40

## Description

### Field of the invention

The invention pertains to a peptide for the treatment of patients with leptin-resistant obesity, Bardet-Biedl syndrome and other ciliopathies, like primary ciliary dyskinesia, polycystic kidney and liver disease, nephronophthisis, Alström syndrome, Meckel-Gruber syndrome and retinal degeneration caused by ciliopathy, which is preferably retinitis pigmentosa, rod-cone disease and retinal neuropathy.

### General Background

EP1012188A discloses N-acetyl-GLP-1(7-34)amide and derivatives thereof for treating diabetes mellitus and obesity.

Bardet-Biedl syndrome (BBS) - which has been named after Georges Bardet and Arthur Biedl - is a ciliopathic human genetic disorder that produces many effects and affects many body systems. It is characterized principally by obesity, retinitis pigmentosa, polydactyly, hypogonadism, and renal failure in some cases.^{[1]} Slower mental processing has also been considered a principal symptom.

Bardet-Biedl syndrome is a pleiotropic disorder with variable expressivity and a wide range of clinical variability observed both within and between families. The main clinical features are rod-cone dystrophy, with childhood-onset visual loss preceded by night blindness; postaxial polydactyly; truncal obesity that manifests during infancy and remains problematic throughout adulthood; specific learning difficulties in some but not all individuals; male hypogenitalism and complex female genitourinary malformations; and renal dysfunction, a major cause of morbidity and mortality. There is a wide range of secondary features that are sometimes associated with BBS including
- Speech disorder/delay
- Strabismus/cataracts/astigmatism
- Brachydactyly/syndactyly of both the hands and feet is common, as is partial syndactyl (most usually between the second and third toes)
- Developmental delay: Many children with BBS are delayed in reaching major developmental milestones including gross motor skills, fine motor skills, and psychosocial skills (interactive play/ability to recognize social cues).
- Polyuria/polydipsia (nephrogenic diabetes insipidus)
- Ataxia/poor coordination/imbalance
- Mild hypertonia (especially lower limbs)
- Diabetes mellitus
- Dental crowding/hypodontia/small dental roots; high-arched palate
- Cardiovascular anomalies
- Hepatic involvement
- Anosmia
- Auditory deficiencies
- Hirschsprung disease
- Eyes: Pigmentary retinopathy, poor visual acuity, low vision, and/or blindness caused by an impaired photoreceptor transport mechanism in the retina.
- Nose: Loss of, or reduced sense of, smell (anosmia). Some patients claim extra-sensitive sense of smell.
- Hand and foot: Polydactyly (extra digits) or syndactyly (webbing of fingers and toes).
- Cardiovascular system: Hypertrophy of interventricular septum and left ventricle and dilated cardiomyopathy.
- Gastrointestinal system: Fibrosis.
- Urogenital system: Hypogonadism, renal failure, urogenital sinuses, ectopic urethra, uterus duplex, septate vagina, and hypoplasia of the uterus, ovaries, and fallopian tubes.
- Growth and development: Developmental delay, especially of fine and gross motor skills
- Behavior: a wide variety of socialization and social interaction problems have been identified with BBS.
- Defective thermosensation or mechanosensation.
- Additional features: Obesity, possibly related to a decreased sensory function that would normally indicate satiation. Hyperphagia in some patients.

The detailed biochemical mechanism that leads to BBS is still unclear.

The gene products encoded by these BBS genes, called BBS proteins, are located in the basal body and cilia of the cell.^{[2]}

Using the round worm *C. elegans* as a model system, biologists found that BBS proteins are involved in a process called intraflagellar transport (IFT), a bi-directional transportation activity within the cilia along the long axis of the ciliary shaft that is essential for ciliogenesis and the maintenance of cilia.^{[3]} Recent biochemical analysis of human BBS proteins revealed that BBS proteins are assembled into a multiple protein complex, called "BBSome". BBSome is proposed to be responsible for transporting intracellular vesicles to the base of the cilia and to play an important role in the ciliary function.

Since abnormalities of cilia are known to be related to a wide range of disease symptoms including those commonly seen in BBS patients, it is now widely accepted that mutated BBS genes affect normal cilia function, which, in turn, causes BBS.

A theory that photoreceptor cells are nourished by the intraflagellar transport of retinal cilia now offers a potential explanation for the retinal dystrophy common in BBS patients after their early years of live.^{[4][5]}

Recent findings in genetic research have suggested that a large number of genetic disorders, both genetic syndromes and genetic diseases, that were not previously identified in the medical literature as related, may be, in fact, highly related in the genetypical root cause of the widely varying, phenotypically observed disorders. BBS is one such syndrome that has now been identified to be caused by defects in the cellular ciliary structure. Thus, BBS is a ciliopathy. Other known ciliopathies include primary ciliary dyskinesia, polycystic kidney and liver disease, nephronophthisis, Alström syndrome, Meckel-Gruber syndrome and some forms of retinal degeneration.^{[6]} Moreover, it is known that leptin inhibits food intake and stimulates weight loss via stimulation of leptin receptors in the arcuate nucleus of the brain^{[7][8]}.

Further, it is known that GLP-1 receptor agonists inhibit food intake and stimulate weight loss via stimulation of GLP-1 receptors in the arcuate nucleus of the brain^{[9]}.

Moreover, it is known that GLP-1 receptor agonists have food intake inhibiting and weight loss stimulating effects only when leptin receptors are present, but not in leptin-resistance where leptin receptors are missing ^{[10]}.

In spite of extensive research, no compound with substantial protective and/or curative potential in patients with Bardet-Biedl syndrome or other ciliopathies has been identified yet. There is therefore an urgent need for novel therapeutic approaches in Bardet-Biedl syndrome and other ciliopathies. The same applies to the prevention and treatment of leptin-resistant obesity and the prevention and treatment of retinal degeneration caused by ciliopathy, in particular retinitis pigmentosa, rod-cone dystrophy and retinal neuropathy.

### Summary of the invention

The object of the present invention is to provide a treatment for patients with leptin-resistant obesity, Bardet-Biedl syndrome and other ciliopathies, to protect such patients from development of pathological symptoms, or to slow down progression of pathological symptoms or to cure pathological symptoms. "Other ciliopathies" in this context are preferably ciliary dyskinesia, polycystic kidney and liver disease, nephronophthisis, Alström syndrome, Meckel-Gruber syndrome.

Surprisingly the inventors have found that GLP-1 receptor agonists inhibit food intake and stimulate weight loss and reduce the progression of rod-cone dystrophy in a mouse model for Bardet-Biedl syndrome with leptin-receptor deficiency. Hence, the present invention provides a treatment for patients with retinal degeneration caused by ciliopathy, which is preferably retinitis pigmentosa, rod-cone disease and retinal neuropathy.

### Detailed description of the invention

The present invention is based on the surprising finding that the compounds of the invention have protective and curative effects on pathological symptoms developing in patients with leptin-resistant obesity, Bardet-Biedl syndrome and in patients with other ciliopathies without simultaneous administration of other compounds, specifically they have protective and/or curative effects on neurodegeneration, diabetes mellitus, obesity, hyperphagia, rod-cone dystrophy and ataxia.

It may be stated that leptin inhibits food intake and stimulates weight loss via stimulation of leptin receptors in the arcuate nucleus of the brain^{[7,8]}. GLP-1 receptor agonists inhibit food intake and stimulate weight loss via stimulation of GLP-1 receptors in the arcuate nucleus of the brain^{[9]}. GLP-1 receptor agonists have food intake inhibiting and weight loss stimulating effects only when leptin receptors are present, but not in leptin-resistance where leptin receptors are missing ^{[10]}.

Surprisingly it was found that GLP-1 receptor agonists such as Ac-GLP-1 (7-34)amide inhibit food intake and stimulate weight loss in a mouse model for Bardet-Biedl syndrome with leptin-receptor deficiency. Also, to our surprise, Ac-GLP-1 (7-34)amide inhibits apoptosis of retinal cells in the same and in another mouse model for Bardet-Biedl syndrome.
(1) In a first embodiment, the invention relates to a peptide of the formula R¹-GLP-1-(7-34)-R² comprising the following sequence:

   R¹-NH-HAEGTFTSDVSSYLEGQAAKEFIAWLVK-C(O)R²

   wherein
   R¹ is H, C₍₁₋₁₀₎-alkyl or C(O)R^{1a} wherein R^{1a} is H or C₍₁₋₉₎-alkyl;
   R² is -OH, or -NR³R⁴, wherein R³ and R⁴ are independently H or C₍₁₋₄₎alkyl; and optionally the following substitutions:
      - A in position 8 is substituted by a neutral amino acid selected from S, S†, G, C, C†, Sar, beta-ala and/or Aib; and/or
      - G in position 10 is substituted by a neutral amino acid selected from S, S†, C, C†, Sar, beta-ala and/or Aib; and/or
      - D in position 15 is substituted by E;
      for the use in the treatment and prophylaxis of ciliopathies, leptin-resistant obesity and/or retinal degeneration caused by ciliopathy.
   The term "substituted by" means "replaced by".
   In the sequence of the peptides the amino acids are symbolized in single letter code, but with explicit designating the N-terminal end (R¹-NH-) and C-terminal end (-C(O)R²).
   The symbol † means the D-form of the respective amino acid. Sar means sarcosine. Beta-A means beta-ala, i.e. beta-alanine. Aib means 2-aminoisobutyric acid or alpha-methylalanine or 2-methylalanine. K-methylalanine-argininamide means lysine-N-methylalanine-argininamide, i.e. a residue of the following formula 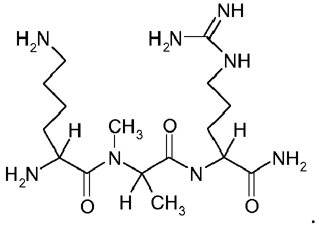 The term "alkyl" means a straight or branched chain alkyl group containing from 1 to 10 carbon atoms. Examples of alkyl are methyl, ethyl, *n*-propyl, *iso*-propyl, *n*-butyl, *iso*-butyl, *sec*-butyl and *tert*-butyl*.*
   As defined herein, the present peptide is for the use in the treatment and prophylaxis of ciliopathies, leptin-resistant obesity and for retinal degeneration caused by ciliopathy. Ciliopathies are in particular selected from Bardet-Biedl syndrome, primary ciliary dyskinesia, polycystic kidney and liver disease, nephronophthisis, Alström syndrome, Meckel-Gruber syndrome and/or retinal degeneration caused by ciliopathy. Retinal degenerations caused by ciliopathy are in particular retinitis pigmentosa, rod-cone dystrophy and retinal neuropathy. Preferably, the ciliopathy is Bardet-Biedl syndrome. As further defined herein, the present peptide is for the use in the treatment and prophylaxis of leptin-resistant obesity.
   The present invention is therefore in particular for the use in the treatment and prophylaxis of Bardet-Biedl syndrome, leptin-resistant obesity and retinal degeneration caused by ciliopathy.
   In one embodiment, the present invention is in particular for the use in the treatment and prophylaxis of Bardet-Biedl syndrome.
   In one embodiment, the present invention is in particular for the use in the treatment and prophylaxis of leptin-resistant obesity.
   In one embodiment, the present invention is in particular for the use in the treatment and prophylaxis of retinal degeneration caused by ciliopathy.
(2) In a further embodiment, the peptide is defined according to embodiment (1), wherein R¹ is H, or C(O)R^{1a} wherein R^{1a} is C₍₁₋₉₎-alkyl, and R² is -NH₂, for the use in the treatment and prophylaxis of ciliopathies, leptin-resistant obesity and/or retinal degeneration caused by ciliopathy.
   The diseases are in particular those as defined in embodiment (1).
(3) In a further embodiment, the peptide is defined according to embodiment (1) or (2), wherein R¹ is C(O)R^{1a} wherein R^{1a} is H, methyl or ethyl, i.e. R¹ is formyl, acetyl or propionyl, for the use in the treatment and prophylaxis of ciliopathies, leptin-resistant obesity and/or retinal degeneration caused by ciliopathy.
   The diseases are in particular those as defined in embodiment (1).
(4) In a further embodiment, the peptide is defined according to embodiment (1) or (3), wherein R³ and R⁴ are independently hydrogen, methyl, ethyl, propyl, isopropyl, n-butyl, sec-butyl or tert-butyl. Preferably, R³ and R⁴ are independently hydrogen, methyl or ethyl for the use in the treatment and prophylaxis of ciliopathies, leptin-resistant obesity and/or for retinal degeneration caused by ciliopathy. It is to be understood that all residues R³ and R⁴ can be mixed with each other.
   The diseases are in particular those as defined in embodiment (1).
(5) In one embodiment, the peptide is defined according to embodiment (1), (2), (3) or (4), wherein the peptide of the formula R¹-GLP-1-(7-34)-R² comprises the following sequence:

   R¹-NH-HAEGTFTSDVSSYLEGQAAKEFIAWLVK-C(O)R²,

   for the use in the treatment and prophylaxis of ciliopathies, leptin-resistant obesity and/or retinal degeneration caused by ciliopathy. The diseases are in particular those as defined in embodiment (1).
(6) In a further embodiment, the peptide of embodiment (1), (2), (3), or (4), relates to the sequence:

   R¹-NH-HAEGTFTSDVSSYLEGQAAKEFIAWLVK-C(O)R²

   wherein R¹ is C(O)CH₃ and R² is NH₂, for the use in the treatment and prophylaxis of ciliopathies and leptin-resistant obesity and/or retinal degeneration caused by ciliopathy. I.e. the sequence is H₃CC(O)NH-HAEGTFTSDVSSYLEGQAAKEFIAWLVK-CONH₂, for the use in the treatment and prophylaxis of ciliopathies, leptin-resistant obesity and/or for retinal degeneration caused by ciliopathy. The diseases are in particular those as defined in embodiment (1).
(7) In a further embodiment, the peptide is defined according to embodiment (1), (2), (3), or (4),
   wherein
   - (γ-E(α-palmitoyl)) is linked to K in position 26, and K in position 34 is substituted by R-G-R-G, R² is OH; or
   - A in position 8 is substituted by G and R² is GR; or
   - A in position 8 is substituted by G, G in position 22 is substituted by E, R² is G; or
   - A in position 8 is substituted by Aib, and R² is AibR-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
   for the use in the treatment and prophylaxis of ciliopathies, leptin-resistant obesity and/or retinal degeneration caused by ciliopathy.
   The diseases are in particular those as defined in embodiment (1).
   Preferably, R¹ is H, C₍₁₋₁₀₎-alkyl or C(O)R^{1a} wherein R^{1a} is H or C₍₁₋₉₎-alkyl;
   More preferably, R¹ is H, formyl, acetyl or propionyl; and even more preferably, R¹ is H.
(8) In particular, the peptide of the formula R¹-GLP-1-(7-34)-R² comprises the following sequence:

   R¹-NH-HAEGTFTSDVSSYLEGQAAKEFIAWLVK-C(O)R²

   wherein
   - (γ-E(α-palmitoyl)) is linked to K in position 26, and K in position 34 is substituted by R-G-R-G, R² is OH; or
   - A in position 8 is substituted by G and R² is GR; or
   - A in position 8 is substituted by G, G in position 22 is substituted by E, R² is G; or
   - A in position 8 is substituted by Aib, and R² is AibR-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
   for the use in the treatment and prophylaxis of ciliopathies, leptin-resistant obesity and/or retinal degeneration caused by ciliopathy.
   The diseases are in particular those as defined in embodiment (1).
   Preferably, R¹ is H, C₍₁₋₁₀₎-alkyl or C(O)R^{1a} wherein R^{1a} is H or C₍₁₋₉₎-alkyl;
   More preferably, R¹ is H, formyl, acetyl or propionyl; and even more preferably, R¹ is H.
(9) In a further embodiment, the peptide is defined according to embodiment (1), wherein
   R¹ is H, formyl, acetyl or propionyl; and
   - (γ-E(α-palmitoyl)) is linked to K in position 26, and K in position 34 is substituted by R-G-R-G, R² is OH; or
   - A in position 8 is substituted by G and R² is GR; or
   - A in position 8 is substituted by G, G in position 22 is substituted by E, R² is G; or
   - A in position 8 is substituted by Aib, and R² is AibR-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
   for the use in the treatment and prophylaxis of ciliopathies, leptin-resistant obesity and/or retinal degeneration caused by ciliopathy.
   The diseases are in particular those as defined in embodiment (1).
(10) In particular, the peptide of the formula R¹-GLP-1-(7-34)-R² comprises the following sequence:

   R¹-NH-HAEGTFTSDVSSYLEGQAAKEFIAWLVK-C(O)R²

   wherein
   R¹ is H, formyl, acetyl or propionyl; and
   - (γ-E(α-palmitoyl)) is linked to K in position 26, and K in position 34 is substituted by R-G-R-G, R² is OH; or
   - A in position 8 is substituted by G and R² is GR; or
   - A in position 8 is substituted by G, G in position 22 is substituted by E, R² is G; or
   - A in position 8 is substituted by Aib, and R² is AibR-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
   for the use in the treatment and prophylaxis of ciliopathies, leptin-resistant obesity and/or retinal degeneration caused by ciliopathy.
   The diseases are in particular those as defined in embodiment (1).
   Preferably, R¹ is H.
(11) In a further embodiment, the peptide is defined according to embodiment (7), (8), (9) or (10), having the sequence:

   R¹-NH-HAEGTFTSDVSSYLEGQAAKEFIAWLVK-C(O)R²

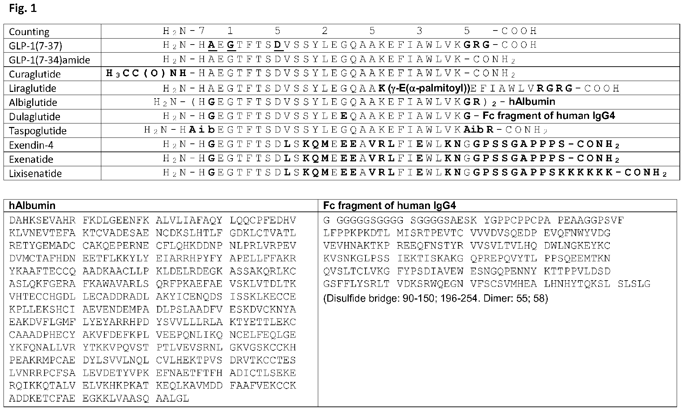

   wherein R¹ is H, and
   - (γ-E(α-palmitoyl)) is linked to K in position 26, and K in position 34 is substituted by R-G-R-G and R² is OH; or
   - A in position 8 is substituted by G and R² is GR, whereby this sequence is repeated once again, and then extended by Sequence No. 6; or
   - A in position 8 is substituted by G, G in position 22 is substituted by E, R² is G, extended by amino acid Sequence No. 9; or
   - A in position 8 is substituted by Aib, and R² is AibR-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
   - V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
   for the use in the treatment and prophylaxis of ciliopathies, leptin-resistant obesity and/or retinal degeneration caused by ciliopathy. The diseases are in particular those as defined in embodiment (1).
   Thereby, Sequence No. 6 and Sequence No. 9 are as defined in the sequence listing, or as defined by "hAlbumin" and/or "Fc fragment of human IgG4" as shown in Figure 1, or in embodiment (16) and /17).
(12) In one embodiment, the peptide is GLP-1(7-37):
   H₂N-HAEGTFTSDVSSYLEGQAAKEFIAWLVKGRG-COOH for the use in the treatment and prophylaxis of ciliopathies, leptin-resistant obesity and/or retinal degeneration caused by ciliopathy. The diseases are in particular those as defined in embodiment (1).
(13) In one embodiment, the peptide is GLP-1(7-34)amide:
   H₂N-HAEGTFTSDVSSYLEGQAAKEFIAWLVK-CONH₂ for the use in the treatment and prophylaxis of ciliopathies, leptin-resistant obesity and/or retinal degeneration caused by ciliopathy. The diseases are in particular those as defined in embodiment (1).
(14) In one embodiment, the peptide is Curaglutide:
   H₃CC(O)NH-HAEGTFTSDVSSYLEGQAAKEFIAWLVK-CONH₂ for the use in the treatment and prophylaxis of ciliopathies, leptin-resistant obesity and/or retinal degeneration caused by ciliopathy. The diseases are in particular those as defined in embodiment (1).
(15) In one embodiment, the peptide is liraglutide (INN):
   H₂N-HAEGTFTSDVSSYLEGOAAK(γ-E(α-palmitoyl))EFIAWLVRGRG-COOH for the use in the treatment and prophylaxis of ciliopathies, leptin-resistant obesity and/or retinal degeneration caused by ciliopathy. The diseases are in particular those as defined in embodiment (1).
(16) In one embodiment, the peptide is albiglutide (INN):
   H₂N-(HGEGTFTSDVSSYLEGQAAKEFIAWLVKGR)₂-hAlbumin. I.e. the sequence HGEGTFTSDVSSYLEGQAAKEFIAWLVKGR is once repeated, and then extended with human albumin, for the use in the treatment and prophylaxis of ciliopathies, leptin-resistant obesity and/or retinal degeneration caused by ciliopathy. The diseases are in particular those as defined in embodiment (1).
      The sequence reads: 
HGEGTFTSDVSSYLEGQAAKEFIAWLVKGRHGEGTFTSDVSSYLEGQAAKEFIAWLVKGR
DAHKSEVAHRFKDLGEENFKALVLIAFAQYLQQCPFEDHVKLVNEVTEFAKTCVADESAE
NCDKSLHTLFGDKLCTVATLRETYGEMADCCAKQEPERNECFLQHKDDNPNLPRLVRPEV
DVMCTAFHDNEETFLKKYLYEIARRHPYFYAPELLFFAKRYKAAFTECCQAADKAACLLP
KLDELRDEGKASSAKQRLKCASLQKFGERAFKAWAVARLSQRFPKAEFAEVSKLVTDLTK
VHTECCHGDLLECADDRADLAKYICENQDSISSKLKECCEKPLLEKSHCIAEVENDEMPA
DLPSLAADFVESKDVCKNYAEAKDVFLGMFLYEYARRHPDYSVVLLLRLAKTYETTLEKC
CAAADPHECYAKVFDEFKPLVEEPQNLIKQNCELFEQLGEYKFQNALLVRYTKKVPQVST
PTLVEVSRNLGKVGSKCCKHPEAKRMPCAEDYLSVVLNQLCVLHEKTPVSDRVTKCCTES
LVNRRPCFSALEVDETYVPKEFNAETFTFHADICTLSEKERQIKKQTALVELVKHKPKAT
KEQLKAVMDDFAAFVEKCCKADDKETCFAEEGKKLVAASQAALGL
(17) In one embodiment, the peptide is dulaglutide (INN). Thereby, the following sequence is liked to a human Ig4-F heavy chain by a small peptide linker:
   H₂N-HGEGTFTSDVSSYLEEQAAKEFIAWLVKG- Fc fragment of human IgG4, for the use in the treatment and prophylaxis of ciliopathies, leptin-resistant obesity and/or retinal degeneration caused by ciliopathy. The diseases are in particular those as defined in embodiment (1).
   The sequence reads: 
HGEGTFTSDVSSYLEEQAAKEFIAWLVKGGGGGGSGGGGSGGGGSAESKYGPPCPPCPA
PEAAGGPSVFLFPPKPKDTLMISRTPEVTCVVVDVSQEDPEVQFNWYVDGVEVHNAKTKP
REEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRLT
VDKSRWQEGNVFSCSVMHEALHNHYTQKSLSLSLG
(18) In one embodiment, the peptide is taspoglutide (INN):

   H₂N-HAibEGTFTSDVSSYLEGQAAKEFIAWLVKAibR-CONH₂

   for the use in the treatment and prophylaxis of ciliopathies, leptin-resistant obesity and/or retinal degeneration caused by ciliopathy. The diseases are in particular those as defined in embodiment (1).
(19) In one embodiment, the peptide is exendin-4 or exenatide (INN):

   H₂N-HGEGTFTSDLSKQMEEEAVRLFIEWLKNGGPSSGAPPPS-CONH₂

   for the use in the treatment and prophylaxis of ciliopathies, leptin-resistant obesity and/or retinal degeneration caused by ciliopathy. The diseases are in particular those as defined in embodiment (1).
(20) In one embodiment, the peptide is lixisenatide (INN):

   H₂N-HGEGTFTSDLSKQMEEEAVRLFIEWLKNGGPSSGAPPSKKKKKK-CONH₂

   for the use in the treatment and prophylaxis of ciliopathies, leptin-resistant obesity and/or retinal degeneration caused by ciliopathy. The diseases are in particular those as defined in embodiment (1).

**Figure 1** shows a comparison of the peptide sequences of GLP-1 (7-37), GLP-1 (7-34)amide, curaglutide, liraglutide, albiglutide, dulaglutide, taspoglutide, exendin-4, exenatide or lixisenatide. The underlined amino acids A in position 8 and/or G in position 10, as exemplified in GLP-1 (7-37), can be substituted by S, S†, G, C, C†, Sar, beta-ala and/or Aib. It is to be understood that these substitutions may also occur in GLP-1 (7-34)amide, curaglutide, liraglutide, albiglutide, dulaglutide, taspoglutide, exendin-4, exenatide or lixisenatide.

Moreover, the underlined amino acid D in position 15, as exemplified in GLP-1 (7-37), can be substituted by E. It is to be understood that these substitutions may also occur in GLP-1 (7-34)amide, curaglutide, liraglutide, albiglutide, dulaglutide, taspoglutide, exendin-4, exenatide or lixisenatide.

### Medical compositions

The peptide according to the present invention may be formulated in combination with a suitable pharmaceutically acceptable carrier. Such carriers are well known to the skilled person and can be readily derived from textbooks of pharmaceutical formulation.

For example the peptide according to the invention may be formulated as solution, powder, tablet, pill, drops, prefilled syringe in a permanent or pulsative release formulation or formulated for subcutaneous, intravenous, intraarterial, peroral, intramuscular, conjunctival, nasal, intranasal, transnasal, transpulmonary, dermal, transdermal, percutaneous, periocular, circumocular, intraocular, intravitreal, retrobulbar or topical administration.

The dosage in which the peptide shall be applied is dependent on various conditions known to the medical practitioner e.g. depending on the status of the patient and the nature of the peptide and/or its formulation. For example, if the peptide is modified at its terminal ends for inducing prolonged residence times the dosage can be smaller than without the modification. In most cases the dosage can be adjusted by the physician according to his or her professional skills.

Typically, the peptide can be applied in a range of from about 0.01 µg/kg body weight to 1 mg/kg body weight per day, in particular from about 0.05 µg/kg body weight to 30 µg/kg body weight per day. Lower ranges are 0.01 µg/kg body weight/day, 0.02 µg/kg body weight/day, 0.03 µg/kg body weight/day, 0.04 µg/kg body weight/day and 0.05 µg/kg body weight/day. Upper ranges are 1 mg/kg body weight/day, 750 µg/kg body weight/day, 500 µg/kg body weight/day, 250 µg/kg body weight/day, 200 µg/kg body weight/day, 100 µg/kg body weight/day, 75 µg/kg body weight/day, 50 µg/kg body weight/day and 30 µg/kg body weight/day/day. It is to be understood that each lower limit can be combined with each of the upper limits disclosed in this paragraph.

Moreover, the peptide can be applied in form of one or more doses daily, weekly or monthly.

The cellular and molecular details of the biological effects of the compounds of the invention are still not fully understood. While not being bound to any hypothesis or theory of modes of action, a probable mechanism of N-Ac-GLP-1-(7-34)-amide action may involve activation of GLP-1 receptors on cells of various tissues, which develop cilia. This receptor activation in turn activates a number of signalling mechanisms inside the cells, one of which is prevention of mitochondrial permeability transition. Under conditions of mitochondrial calcium overload, the opening of mitochondrial permeability transition pores enables free passage of calcium and other molecules of < 1.5 kDa into the mitochondria including protons. The resulting uncoupling of oxidative phosphorylation leads to adenosine triphosphate (ATP) depletion and subsequently to necrotic cell death.

### Experimental part

**Experiment 1a:** Effects of GLP-1 in ex-vivo model (retinal explants): In BBS12-deprived retinal explants and the *Bbs12*^{/} murine model, impaired intraciliary transport results in protein retention in the endoplasmic reticulum. The protein overload activates a pro-apoptotic unfolded protein response leading to a specific Caspase12-mediated apoptosis of the photo-receptors. BBS12-deprived retinal explants are incubated with Ac-GLP-1 (7-34)amide and Caspase12-mediated apoptosis of the photo-receptors is compared to the apoptosis rate of BBS12-deprived retinal explants, which have been treated with the vehicle only.

### Methods; see [11]

- 15-day-old mice are sacrificed by decapitation, and the eyes are removed
- First, the sclera is carefully removed, leaving the retinal pigmented epithelium attached to the eye ball.
- Incisions are made at the edge of the cornea to remove cornea, lens, and hyaloid body.
- Retinas are transferred, with retinal pigmented epithelium side down, to a culture membrane
- Retina explants are maintained at 37 °C in a humidified 5% CO2 atmosphere.
- Specific gene silencing with lentiviruses that carry a shRNA sequence for Bbs12 is performed.
- Drugs are added to the culture medium

**Experiment 1b:** Effects of Ac-GLP-1 (7-34)amide in in-vivo-model (BBS12 mice): BBS12^{-/-} mice are housed in humidity- and temperature-controlled rooms on a 12-h light/12-h dark cycle with food and water *ad libitum.* Ac-GLP-1 (7-34)amide administration is applied systemically by s.c. application or in form of eye drops in watery solution.

Eye drop treatment is given between 2 and 4 weeks of age, and systemic treatment is given between 3 and 4 weeks of age. At 4 weeks of age, electrophysiological analyses are performed, and the retinas are harvested for molecular analysis.

**Experiment 2:** The purpose of the experiment is to study the effects of GLP-1 receptor agonists on
- body weight and food intake of leptin-resistant obese mice carrying the M390R mutation in the Bbs1 gene (Bbs1^{M390R}) and on
- body weight and food intake of lean wild type littermate controls, both groups fed *ad libitum.* In this particular experiment the GLP-1 receptor agonist used is acetyl-GLP-1(7-34)amide. The M390R mutation in the Bbs1 gene can also be found in human Bardet-Biedl syndrome.

Male 12-16 weeks old mice are housed in individual cages for 3-5 days to acclimate to single housing conditions before baseline body weight and 24-hour food intake is determined during 4 days. Then food intake and body weight is tested in response to treatment with acetyl-GLP-1(7-34)amide. Mice are treated intraperitoneally with vehicle or with the GLP-1 receptor agonist twice per day (9 am and 5 pm) for 7 days as follows:
Group 1 - Lean control mice treated with vehicle (n=10)
Group 2 - Lean control mice treated with the GLP-1 receptor agonist in a dosing volume of 0.1 mL (n=10).
Group 3 - Obese Bbs1^{M390R} mice treated with vehicle (n=10)
Group 4 - Obese Bbs1^{M390R} mice treated with the GLP-1 receptor agonist in a dosing volume of 0.1 mL (n=10)

During the treatment period, mice continue to have free access to food. Measurement of body weight and 24-hour food intake continues during the treatment. At the end of the 7-day treatment period, body composition is determined in live mice using nuclear magnetic resonance (NMR) tomography. Mice are then sacrificed and the weight of various organs and tissues including various fat depots, liver, kidneys, heart, lung and brain are measured. Blood is obtained from every mouse to measure glucose. Plasma is stored in minus 80°C for future measurement of plasma leptin and GLP-1.

**Experiment 3:** The purpose of the experiment is to study the effects of GLP-1 receptor agonists on
- Development of rod-cone dystrophy in mice carrying the M390R mutation in the Bbs1 gene (Bbs1^{M390R}). In this particular experiment the GLP-1 receptor agonist used is acetyl-GLP-1(7-34)amide. The M390R mutation in the Bbs1 gene can also be found in human Bardet-Biedl syndrome.

Male Bbs1^{M390R} mice are used in the experiment. From postnatal day 6 to postnatal day 30 the experimental group is treated intraperitoneally with acetyl-GLP-1 (7-34)amide. Mice of the control group are treated during the same age period with vehicle.

Analysis of endpoints: comparison of control vs GLP-1
At the end of the treatment period, mice are investigated by use of electroretinography (ERG), optical coherence tomography (OCT) and histology.

### References

[1] Beales P, Elcioglu N, Woolf A, Parker D, Flinter F (1 June 1999). "New criteria for improved diagnosis of Bardet-Biedl syndrome: results of a population survey". J. Med. Genet. 36 (6): 437-46.
[2] Ansley SJ, Badano JL, Blacque OE, Hill J, Hoskins BE, Leitch CC, Kim JC, Ross AJ, Eichers ER, Teslovich TM, Mah AK, Johnsen RC, Cavender JC, Lewis RA, Leroux MR, Beales PL, Katsanis N (October 2003). "Basal body dysfunction is a likely cause of pleiotropic Bardet-Biedl syndrome". Nature. 425 (6958): 628-33
[3] Blacque OE, Reardon MJ, Li C, McCarthy J, Mahjoub MR, Ansley SJ, Badano JL, Mah AK, Beales PL, Davidson WS, Johnsen RC, Audeh M, Plasterk RH, Baillie DL, Katsanis N, Quarmby LM, Wicks SR, Leroux MR (2004). "Loss of C. elegans BBS-7 and BBS-8 protein function results in cilia defects and compromised intraflagellar transport". Genes Dev. 18 (13): 1630-42.
[4] Sedmak T, Wolfrum U (April 2010). "Intraflagellar transport molecules in ciliary and nonciliary cells of the retina". J. Cell Biol. 189 (1): 171-86.
[5] Orozco, JT; Wedaman KP; Signor D; Brown H; Rose L; Scholey JM (1999). "Movement of motor and cargo along cilia". Nature. 398 (6729): 674.
[6] Badano JL, Mitsuma N, Beales PL, Katsanis N (2006). "The ciliopathies: an emerging class of human genetic disorders". Annu Rev Genomics Hum Genet. 7: 125-48.
[7] Rahmouni K, et al.; Leptin resistance contributes to obesity and hypertension in mouse models of Bardet-Biedl syndrome. J. Clin. Invest. 118:1458-1467 (2008).
[8] Guo D-F, et al; The BBSome controls energy homeostasis by mediating the transport of the leptin receptor to the plasma membrane. PLOS Genetics 12 (2) Feb. 29, 2016
[9] Baggio LL, et al; Glucagon-like peptide-1 receptors in the brain: controlling food intake and body weight. J Clin Invest. 2014;124(10):4223-4226
[10] Williams DL et al.: Leptin regulation of the anorexic response to Glucagon-like peptide-1 receptor stimulation. Diabetes 55: 3387-3393, 2006
[11] Mockel A. et al.; Pharmacological modulation of the retinal unfolded protein response in Bardet-Biedl syndrome reduces apoptosis and preserves light detection ability. J.Biol.Chem. 287 (44), 37483-37494, 2012.

## Claims

1. A peptide of the formula R¹-GLP-1-(7-34)-R² comprising the following sequence:
R¹-NH-HAEGTFTSDVSSYLEGQAAKEFIAWLVK-C(O)R²
wherein
R¹ is H, C₍₁₋₁₀₎-alkyl or C(O)R^{1a} wherein R^{1a} is H or C₍₁₋₉₎-alkyl;
R² is -OH, or -NR³R⁴, wherein R³ and R⁴ are independently H or C₍₁₋₄₎alkyl; and
optionally the following substitutions:
- A in position 8 is substituted by a neutral amino acid selected from S, S†, G, C, C†, Sar, beta-ala and/or Aib; and/or
- G in position 10 is substituted by a neutral amino acid selected from S, S†, C, C†, Sar, beta-ala and/or Aib; and/or
- D in position 15 is substituted by E;
for the use in the treatment and prophylaxis of ciliopathies, leptin-resistant obesity and/or retinal degeneration caused by ciliopathy.

2. The peptide of claim 1, wherein
R¹ is H, or C(O)R^{1a} wherein R^{1a} is C₍₁₋₉₎-alkyl, and R² is -NH₂,
for the use in the treatment and prophylaxis of ciliopathies, leptin-resistant obesity and/or retinal degeneration caused by ciliopathy.

3. The peptide of claim 2, having the sequence:
R¹-NH-HAEGTFTSDVSSYLEGQAAKEFIAWLVK-C(O)R²
wherein R¹ is C(O)CH₃ and R² is NH₂, for the use in the treatment and prophylaxis of ciliopathies, leptin-resistant obesity and/or retinal degeneration caused by ciliopathy.

4. The peptide of claim 1, wherein
- (γ-E(α-palmitoyl)) is linked to K in position 26, and K in position 34 is substituted by R-G-R-G, R² is OH; or
- A in position 8 is substituted by G, R² is GR; or
- A in position 8 is substituted by G, G in position 22 is substituted by E, R² is G; or
- A in position 8 is substituted by Aib, and R² is AibR-CONH₂; or
- V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
- V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
for the use in the treatment and prophylaxis of ciliopathies, leptin-resistant obesity and/or retinal degeneration caused by ciliopathy.

5. The peptide of claim 4, having the sequence:
R¹-NH-HAEGTFTSDVSSYLEGQAAKEFIAWLVK-C(O)R²
wherein R¹ is H, and
- (γ-E(α-palmitoyl)) is linked to K in position 26, and K in position 34 is substituted by R-G-R-G and R² is OH; or
- A in position 8 is substituted by G and R² is GR, whereby this sequence is repeated once again, and then extended by h-albumin / Sequence No. 6; or
- A in position 8 is substituted by G, G in position 22 is substituted by E, R² is G, extended by amino acid Sequence No. 9; or
- A in position 8 is substituted by Aib, and R² is AibR-CONH₂; or
- V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-P-S-CONH₂; or
- V in position 16 is substituted by L; S-Y-L in position 18 to 20 is substituted by K-Q-M; G-Q in position 22 to 23 is substituted by E-E; A-K-E in position 25 to 27 is substituted by V-R-L; A in position 30 is substituted by E; V-K in position 33 to 34 is substituted by K-N-G-G-P-S-S-G-A-P-P-S-K-K-K-K-K-K-CONH₂;
for the use in the treatment and prophylaxis of ciliopathies, leptin-resistant obesity and/or retinal degeneration caused by ciliopathy.

6. The peptide according to any one of claims 1 to 5, for the use defined in claim 1, wherein the ciliopathies are selected from Bardet-Biedl syndrome, primary ciliary dyskinesia, polycystic kidney and liver disease, nephronophthisis, Alström syndrome, Meckel-Gruber syndrome, and the retinal degeneration caused by ciliopathy is selected from retinitis pigmentosa, rod-cone disease and/or retinal neuropathy.

7. The peptide according to any one of claims 1 to 5, for the use defined in claim 1, wherein the ciliopathy is Bardet-Biedl syndrome.

8. A pharmaceutical composition comprising as active ingredient the peptide of any one of claims 1 to 5 and at least one therapeutically inert carrier, for the use in the treatment and prophylaxis of ciliopathies, leptin-resistant obesity and/or retinal degeneration caused by ciliopathy.

9. The peptide according to any one of claims 1 to 5 for the use in the manufacture of a medicament for the use in the treatment and prophylaxis of ciliopathies, leptin-resistant obesity and/or retinal degeneration caused by ciliopathy.

10. A method of treatment and prophylaxis of ciliopathies, leptin-resistant obesity and/or retinal degeneration caused by ciliopathy, comprising administering to a patient an effective amount of the peptide of any one of claim 1 to 5.
